# EUROPEAN PATENT APPLICATION

(11) **EP 4 740 806 A1**
(43) Date of publication of application: **13.05.2026**
(21) Application number: 24845781.4
(22) Date of filing: 03.06.2024
(51) Int. Cl.: A47K 7/04, C25B 11/00, C25B 1/04, B01F 23/2373, C02F 1/467, C02F 1/461, A61L 2/18

(54) **MICROBUBBLE CLEANSING DEVICE COMPRISING ELECTROLYSIS TUBE**

(30) Priority: 21.07.2023 KR 20230095163
(71) Applicant: APR CO., LTD., Seoul 05551 (KR)
(72) Inventor: JI, Jong Chul, Seoul 05551 (KR); LEE, Gyoun Jung, Seoul 05551 (KR); PARK, Seung Woo, Seoul 05551 (KR)
(74) Representative: Henkel & Partner mbB
(86) International application number: PCT/KR2024/007541
(87) International publication number: WO 2025/023477

(57) **Abstract**

A microbubble cleanser having an electrolysis tube capable of providing water containing microbubbles to a user's skin by electrolyzing water to generate microbubbles is disclosed. The microbubble cleanser includes: a water tank (20) coupled to one end of a housing (10) and containing pure water, which is a medium in which microbubbles are generated and supplied; a pump (30) located at the upper part of the water tank (20), withdrawing the water contained in the water tank (20), and transferring the withdrawn water at a predetermined pressure so that the withdrawn water is sprayed onto the user's skin through an electrolysis tube (40); and the electrolysis tube (40) located at one end of the pump (30), electrolyzing the water supplied from the water tank (20) by the pump (30) to generate microbubbles, and supplying the water containing the generated microbubbles to the user's skin.

## Description

### [Technical Field]

The present invention relates to a microbubble cleanser having an electrolysis tube, and more particularly, to a microbubble cleanser having an electrolysis tube capable of electrolyzing water to generate microbubbles and providing water containing microbubbles to a user's skin.

### [Background Art]

Nano or microbubble water is currently expected to be utilized in various fields, such as food (sterilization), beverages, cosmetics, liquid crystal manufacturing, medical (sterilization, cell storage, antibacterial action), aquatic resources or plant cultivation (dissolved oxygen supply, growth promotion), cleaning of electronic device substrates, and reduction of frictional resistance on marine hulls. Generally, microbubbles are generated by applying a voltage to induce discharge between a negative electrode and a positive electrode.

Various configurations of nanobubble production devices have been proposed. Japanese Patent Laid-Open Publication No. 2013-34958 discloses dividing a liquid, mixing one of the divided parts with gas to generate microbubbles, rejoining the divided parts, and supplying the rejoined liquid to a plate with holes and a collision plate provided adjacent thereto to produce nanobubbles.

The present invention discloses miniaturizing an electrolysis tube that generates microbubbles and applying it to a device for skin beauty, specifically for cleansing the skin. The reason why skin cleansing or facial cleansing is important in skin care is that the skin is more exposed to various pollutants than in the past. Although invisible to the human eye, various pollutants exist in the atmosphere, and among them, fine dust and ultrafine dust may not be removed by simple methods such as general hand washing with water.

Various beneficial substances used on the skin to treat, protect, or improve the skin exhibit the best effects when applied to the skin in various ways and then removed at the appropriate time. In addition, these substances must be properly cleansed to avoid adversely affecting the skin. Substances that remain on the skin without being cleansed after time has passed combine with the aforementioned environmental pollutants, adversely affecting the skin, and are not easy to cleanse.

Above all, facial cleansing, which is the process of cleansing the face, must be performed more carefully due to the characteristics of facial skin. Various electrical, chemical, and physical methods have been developed to date for effective and safe facial cleansing. However, there are not many safe and clean cleansing methods that do not damage the skin in the long term.

Patent No. 10-0787874 "Skin Care Device" discloses a massage device having various functions. The device is disclosed to selectively generate ultrasound, infrared rays, ultraviolet rays, etc., to achieve beauty effects through sterilization, treatment, endocrine gland stimulation, blood circulation improvement, etc., but its practical utility and theoretical basis are uncertain.

The formation process of microbubbles is complex, and there is difficulty in miniaturizing the manufacturing device. Meanwhile, there is an increasing demand from consumers to easily and inexpensively perform beauty care or beauty treatments that require a lot of time and cost investment at home at desired times. Therefore, self-treatment type beauty techniques and advanced beauty devices are being steadily developed. However, the technology applied to products still does not meet users' expectations.

### [Prior Art Documents]

(Patent Document 1) International Publication No. WO 2014-148397 A1
(Patent Document 2) Korean Patent No. 10-1383816
(Patent Document 3) Korean Patent Laid-Open Publication No. 10-2013-0127948
(Patent Document 4) Japanese Patent Laid-Open Publication No. 2013-34958

### [Disclosure]

### [Technical Problem]

An object of the present invention is to provide a microbubble cleanser having an electrolysis tube that progressively electrolyzes water to generate microbubbles.

Another object of the present invention is to provide a microbubble cleanser having an electrolysis tube, in which water is introduced into one end of electrodes, and during the process of water flowing out from the other end of the electrodes and being discharged to the outside, the introduced water passes between a pair of electrodes, thereby being progressively electrolyzed to generate microbubbles, thereby easily providing water containing microbubbles to a user's skin at home.

### [Technical Solution]

To achieve the above objects, the present invention provides a microbubble cleanser including: a water tank (20) coupled to one end of a housing (10) and containing pure water, which is a medium in which microbubbles are generated and supplied; a pump (30) located at the upper part of the water tank (20), withdrawing the water contained in the water tank (20), and transferring the withdrawn water at a predetermined pressure so that the withdrawn water is sprayed onto the user's skin through an electrolysis tube (40); and the electrolysis tube (40) located at one end of the pump (30), electrolyzing the water supplied from the water tank (20) by the pump (30) to generate microbubbles, and supplying the water containing the generated microbubbles to the user's skin, wherein the electrolysis tube (40) includes an inlet (46) through which water is supplied, a moving tube (41) through which the water supplied through the inlet (46) moves a predetermined distance, an outlet (48) through which the water moved through the moving tube (41) is discharged, and a pair of electrodes (42) consisting of a first electrode (43) and a second electrode (44) positioned opposite each other on both sides of the moving path of the water moving through the moving tube (41), and applying a voltage to the moving water to progressively electrolyze the water moving through the moving tube (41) to generate microbubbles.

### [Advantageous Effects]

The microbubble cleanser having an electrolysis tube according to the present invention allows water to be introduced into one end of electrodes, and during the process of water flowing out from the other end of the electrodes and being discharged to the outside, the introduced water passes between a pair of electrodes while being progressively electrolyzed to generate microbubbles, thereby providing water containing microbubbles to a user's skin.

### [Description of Drawings]

FIGS. 1 and 2 are respectively a perspective view of the external appearance and a longitudinal sectional view of a microbubble cleanser according to an embodiment of the present invention.
FIG. 3 is an exploded view of a microbubble cleanser according to an embodiment of the present invention.
FIG. 4 is a sectional view showing the structure of a pump that can be used in a microbubble cleanser according to an embodiment of the present invention.
FIG. 5 is a view showing the internal structure of the pump shown in FIG. 4.
FIG. 6 is a view showing an electrolysis tube according to an embodiment of the present invention.
FIG. 7 is a view showing the structure of an electrolysis tube including electrodes according to an embodiment of the present invention.
FIG. 8 is a view showing a moving tube of an electrolysis tube according to an embodiment of the present invention.
FIG. 9 is a photograph showing an actual electrode that can be used in an electrolysis tube according to an embodiment of the present invention.
FIG. 10 is a view showing plating layers of an electrode that can be used in an electrolysis tube according to an embodiment of the present invention.

### [Mode of Disclosure]

Hereinafter, the present invention will be described in detail with reference to the accompanying drawings. In the accompanying drawings, for convenience of explanation, some components may be shown larger or smaller than actual. In addition, in the description of the present invention, detailed descriptions of known general functions or configurations are omitted.

The microbubble cleanser including an electrolysis tube according to the present invention includes: a water tank (20) coupled to one end of a housing (10) and containing pure water, which is a medium in which microbubbles are generated and supplied; a pump (30) located at the upper part of the water tank (20), withdrawing the water contained in the water tank (20), and transferring the withdrawn water at a predetermined pressure so that the withdrawn water is sprayed onto the user's skin through an electrolysis tube (40); and the electrolysis tube (40) located at one end of the pump (30), electrolyzing the water supplied from the water tank (20) by the pump (30) to generate microbubbles, and supplying the water containing the generated microbubbles to the user's skin, and further includes a vibration driving unit (50), a control unit (60), and a vibration cleansing unit (70). The electrolysis tube (40) includes an inlet (46) through which water is supplied, a moving tube (41) through which the water supplied through the inlet (46) moves a predetermined distance, an outlet (48) through which the water moved through the moving tube (41) is discharged, and a pair of electrodes (42) consisting of a first electrode (43) and a second electrode (44) positioned opposite each other on both sides of the moving path of the water moving through the moving tube (41), and applying a voltage to the moving water to progressively electrolyze the water moving through the moving tube (41) to generate microbubbles.

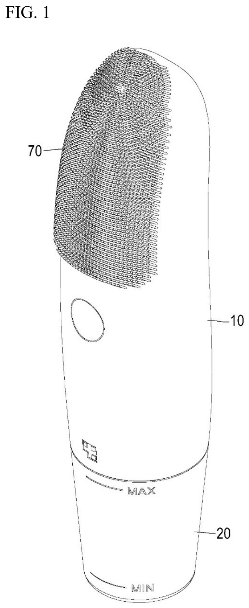

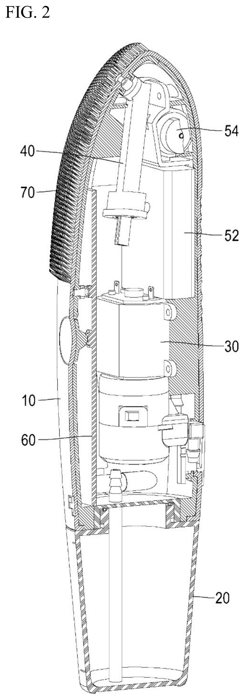

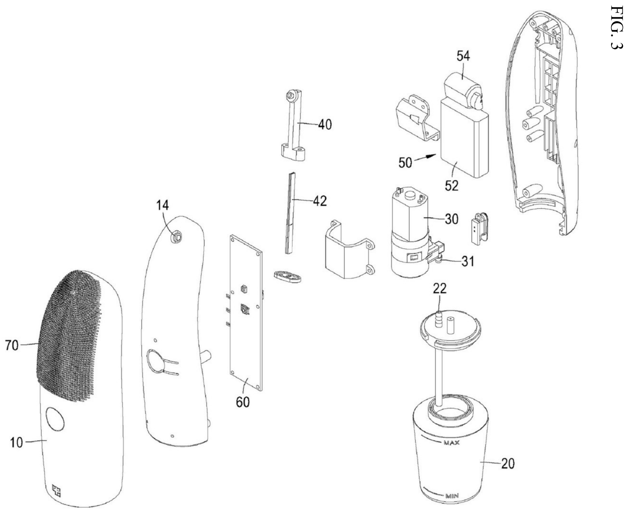

FIGS. 1 and 2 are respectively a perspective view of the external appearance and a longitudinal sectional view of a microbubble cleanser according to an embodiment of the present invention. As shown in FIGS. 1 and 2, the housing (10) may be provided in a substantially cylindrical shape so that the user can hold the microbubble cleanser by hand or use it by attaching a handle. The water tank (20), the pump (30), the electrolysis tube (40), the vibration driving unit (50), the control unit (60), the vibration cleansing unit (70), etc., may be accommodated inside the housing (10) or mounted at one end of the housing (10). FIG. 3 is an exploded view of a microbubble cleanser according to an embodiment of the present invention. As shown in FIG. 3, the vibration cleansing unit (70) is coupled to the upper end of the housing (10), the water tank (20) is coupled to the lower end, and the pump (30), the electrolysis tube (40), the vibration driving unit (50), and the control unit (60) may be mounted inside. A through-hole (14) through which the outlet (48) of the electrolysis tube (40) is exposed to the outside is further formed at the upper end of the housing (10). The through-hole (14) is for discharging water containing microbubbles generated in the electrolysis tube (40) described later to the user's skin. The button located in the middle of the housing (10) is a button for driving the microbubble cleanser. The housing (10) may be made of conventional materials such as hard synthetic resin such as plastic, synthetic resin having a predetermined elasticity such as silicone or rubber, or metal.

The water tank (20) is a container containing water, which is a medium in which microbubbles are generated and supplied, and is coupled to one end of the housing (10), for example, detachably coupled to the lower end of the housing (10). The water as the medium is preferably pure water, not a specially prepared solution.

A water cup nozzle is located at the upper part of the water tank (20). The water cup nozzle and the water tank (20) may be coupled by a conventional method, for example, a plurality of grooves are formed, and they may be coupled by rotating so that the grooves engage, or coupled by fitting. A coupling part coupled to the pump (30) and a nozzle tube (22) connected to the first flow path tube (31) by the tube (34) of the pump (30) may be further formed at the upper part of the water cup nozzle. That is, the water contained in the water tank (20) is moved upward to the pump (30) through the nozzle tube (22) by the pressure of the pump (30).

The pump (30) withdraws the water contained in the water tank (20) and causes the withdrawn water to be sprayed onto the user's skin through the electrolysis tube (40). That is, the pump (30) transfers the water at a predetermined pressure and pushes it toward the user's skin. That is, the water contained in the water tank (20) is supplied from the water tank (20) to the electrolysis tube (40) by the metering pump (30). However, in the longitudinal sectional view shown in FIG. 2, the coupling method of the water tank (20), pump (30), and electrolysis tube (40) is not specifically shown, but the coupling structure will be described later. The pump (30) may be coupled and fixed inside the housing (10) by a predetermined bracket.

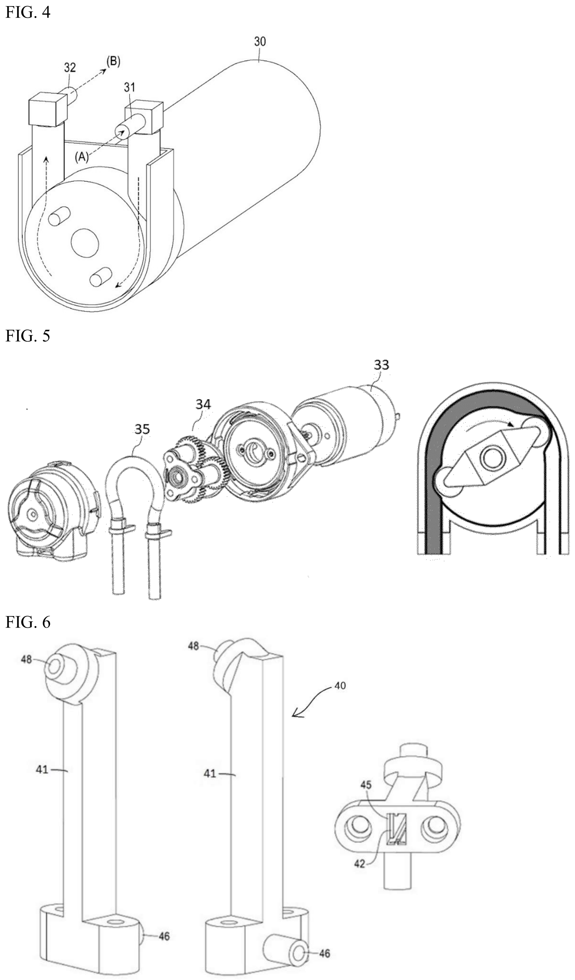

FIG. 4 is a view showing the structure of a pump that can be used in a microbubble cleanser according to an embodiment of the present invention. As shown in FIG. 4, a tube-linked metering pump (Peristaltic Pump) (also referred to as a tubing pump hereinafter, see the example below) is located in the central part of the device. The pump (30) includes a first flow path tube (31) connected to the water tank (20) (specifically, the nozzle tube (22) of the water tank (20)) and a second flow path tube (32) connected to the electrolysis tube (40) (specifically, the inlet (46) of the electrolysis tube (40)). When air is supplied at a predetermined pressure from the pump (30) to the water tank (20) through the first flow path tube (31), the water withdrawn from the water tank (20) through the first flow path tube (31) is transferred to the electrolysis tube (40) at a predetermined pressure through the second flow path tube (32) (the arrow going from (A) to (B) in FIG. 4 indicates the flow of water).

FIG. 5 is a view showing the internal structure of the pump shown in FIG. 4. As shown in FIG. 5, the pump (30) is a tube-linked metering pump, and may include: a motor (33) for driving the pump (30); a plurality of rotating gears located at one end of the motor (33) and rotated by the driving of the motor (33); and tubing (35) surrounding the outer circumferential surface of the plurality of rotating gears, through which the water withdrawn from the water tank (20) moves, that is, a pump driving unit. The tubing (35) is in the form of a hollow tube, and water moves inside the tubing (35). The material of the tubing (35) can be used without limitation.

The driving method of the pump (30) is as follows. For example, when the motor (33) in the pump (30) is driven, the rotating gear (34) is rotated by the rotation shaft of the motor (33), generating a predetermined pressure inside the tubing (35). Accordingly, the water contained in the water tank (20) is withdrawn toward the pump (30) through the first flow path tube (31) connected to the nozzle tube of the water tank (20). Depending on the rotation direction of the rotating gear (34), the water withdrawn to the water tank (20) moves through the tubing (35) and is transferred to the electrolysis tube (40) through the second flow path tube (32) connected to the inlet (46) of the electrolysis tube (40) described later.

The metering pump used as the pump (30) can precisely control the amount of incoming fluid. Using the tubing (35) located inside the pump (30), liquid (for example, water) can be moved without concern for contamination. Therefore, in the process of supplying (transporting) water, the water flowing into the tubing (35) in the pump (30) is not contaminated by the driving device, enabling the supply of clean water suitable for facial cleansing.

The electrolysis tube (40) has a long rectangular parallelepiped shape and is located at one end of the pump (30). The electrolysis tube (40) is a microbubble generator that progressively electrolyzes a portion of the water supplied from the water tank (20) by the pump (30) to generate microbubbles, and supplies the water containing the generated microbubbles to the user's skin.

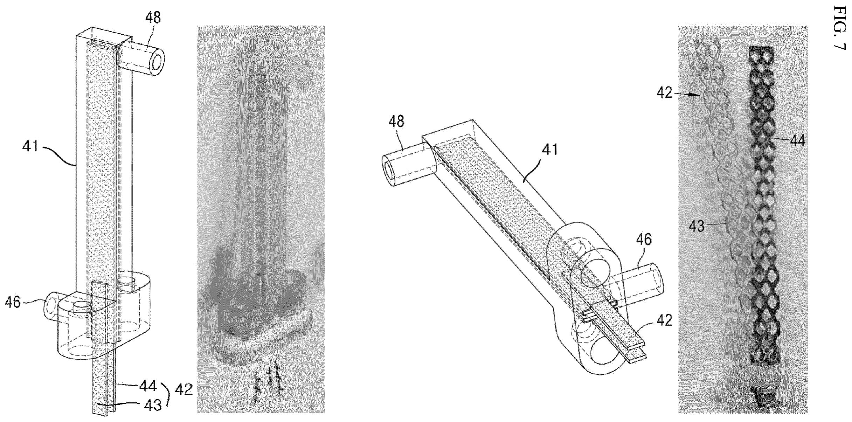

FIG. 6 is a view showing an electrolysis tube according to an embodiment of the present invention, and FIG. 7 is a view showing the structure of an electrolysis tube including electrodes according to an embodiment of the present invention. As shown in FIGS. 6 and 7, the electrolysis tube (40) includes: an inlet (46) through which water is supplied; a moving tube (41) through which the water supplied through the inlet (46) moves a predetermined distance; an outlet (48) through which the water moved through the moving tube (41) is discharged; and a pair of electrodes (42) consisting of a first electrode (43) and a second electrode (44) positioned opposite each other at a predetermined interval on both sides of the moving path of the water moving through the moving tube (41), and applying a voltage to the moving water to progressively electrolyze the water moving through the moving tube (41) to generate microbubbles. The electrolysis tube (40) has a rectangular parallelepiped shape and is a microbubble generator that electrolyzes a portion of the water supplied from the inlet (46) to generate microbubbles.

The inlet (46) is connected to the pump (30), specifically one end (first flow path tube (31)) of the tube (35) located inside the pump (30), so that the water contained in the water tank (20) is supplied (withdrawn) to the electrolysis tube (40) by a predetermined pressure. The outlet (48) exists at the same position as the through-hole (14) of the housing and the through-hole (74) of the vibration cleansing unit described later, so that the water in which microbubbles are generated can be discharged (sprayed) to the user's skin.

The water supplied from the inlet (46) of the electrolysis tube (40) flows along the moving tube (41), and at this time, by the pair of electrodes (42) located inside the moving tube (41), a portion of the water is electrolyzed to generate microbubbles, thereby forming water containing microbubbles.

Specifically, the water supplied from the inlet (46) flows along the moving tube (41). A portion of the water moving through the moving tube (41) of the electrolysis tube (40) passes between the first electrode (43) and the second electrode (44). At this time, when a voltage is applied to the first electrode (43) and the second electrode (44), a portion of the water moving between the first electrode (43) and the second electrode (44) is electrolyzed. Specifically, the first electrode (43) and the second electrode (44) are electrically connected to an external power source, for example, a battery (52). More specifically, when current is supplied from the external power source to the first electrode (43) and the second electrode (44), underwater discharge occurs in the entire area between the two electrodes, active electrolysis of water molecules occurs, and microbubbles are generated. Therefore, the water supplied from the inlet (46) of the electrolysis tube is electrolyzed until the end when it is discharged through the outlet (48), progressively generating microbubbles. The water containing microbubbles is discharged through the outlet (48) of the electrolysis tube (40). In addition, a portion of the water electrolyzed in the electrolysis tube (40) is heated, so that lukewarm water rich in microbubbles can be applied to the user's skin.

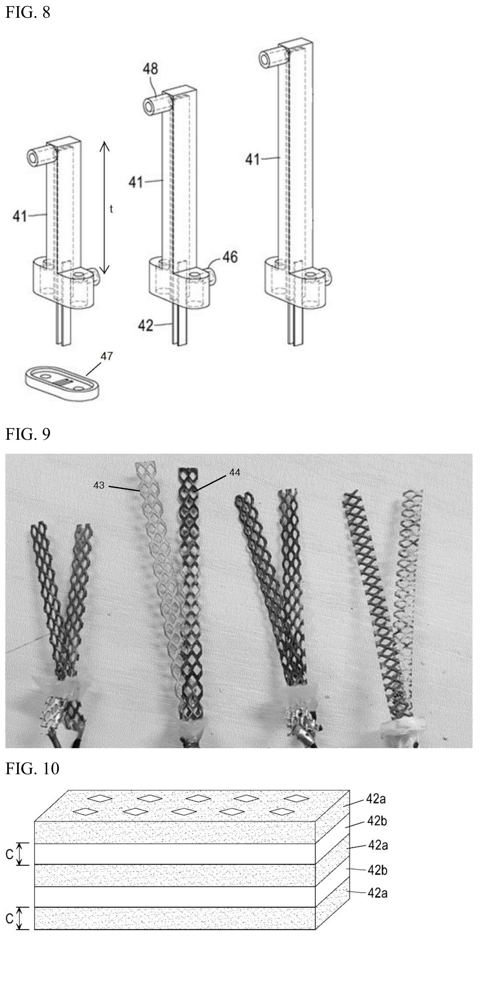

The pair of electrodes (42) is fixed and connected to the moving tube (41) by a module cap (47, see FIG. 8). Specifically, the module cap (47) is fitted and coupled to the lower part of the moving tube (41). After the first electrode (43) and the second electrode (44) are positioned inside the moving tube (41), when the module cap (47) is coupled to the lower part of the moving tube (41), the first electrode (43) and the second electrode (44) are fixed inside the moving tube (41).

The pair of electrodes (42) is fixed inside the moving tube by a fixing guide (45, see FIG. 6, which may be a fixing rail or the like) on the inside. The fixing guide (45) may be structurally varied in various ways.

FIG. 8 is a view showing a moving tube of an electrolysis tube according to an embodiment of the present invention. As shown in FIG. 8, during the process in which the water supplied from the inlet (46) is discharged through the outlet (48), the water is progressively electrolyzed to generate microbubbles. The moving distance of the water moving through the moving tube (41) is related to the length (t) of the first electrode (43) and the second electrode (44) located in the moving tube (41).

The water moving through the moving tube (41) moves along the length direction of the electrodes (42). That is, the water supplied to the inlet (46) is introduced into one end (lower end of the electrodes (42)) of the electrodes (42), progressively electrolyzed while passing between the pair of electrodes (42), and flows out to the other end (upper end of the electrodes (42)). One end (lower end) of the first electrode (43) and the second electrode (44) is located close to the inlet (46), so that the water supplied from the inlet (46) is introduced. The other end (upper end) of the first electrode (43) and the second electrode (44) is located close to the outlet (48), so that the microbubbles generated by the electrolysis flow out to the outlet (48).

Therefore, the moving distance of the water moving through the moving tube (41) is substantially the same as the length (t) of the first electrode (43) and the second electrode (44) that apply voltage to the water moving through the moving tube (41). Here, substantially the same means that the length (t) of the first electrode (43) and the second electrode (44) that apply voltage to the water moving through the moving tube (41) is 90% or more, preferably 95% or more, more preferably 100% of the moving distance of the water moving through the moving tube (41). Therefore, the length (t) of the first electrode (43) and the second electrode (44) located in the moving tube (41) is 90% or more, preferably 95% or more, more preferably substantially the same as the length from the inlet (46) to the outlet (48) of the moving tube (41).

Although the microbubbles stay in water longer than general bubbles, the microbubbles must be fully applied to the skin to effectively affect cleansing. Therefore, the interval between the time when microbubbles are generated from the microbubble generator and the time when they are applied to the skin should be minimized. Therefore, the moving distance of the water moving through the moving tube (41) is substantially the same as the length (t) of the first electrode (43) and the second electrode (44) that apply voltage to the water moving through the moving tube (41).

The length of the moving tube (41) of the electrolysis tube (40), that is, the length (t) of the first electrode (43) and the second electrode (44) that apply voltage to the water moving through the moving tube (41), can be adjusted. That is, the length of the moving tube (41) can be manufactured to a desired length. In this way, the size of the electrolysis tube (40) can be adjusted, and it can be manufactured not only as an ultra-small electrolysis tube but also large electrolysis tube. The electrolysis tube (40) can be used for various purposes, for example, in various applications such as household cleansing devices, water purifiers, water softeners, etc.

If the area such as the length of the first electrode and the second electrode is large, and the interval between the first electrode and the second electrode facing each other is narrow, the efficiency of electrolysis is high, so more microbubbles can be generated. However, the consumption of current applied to the first electrode and the second electrode increases, and if the electrolysis efficiency increases due to the generated microbubbles, the metals plated on the first electrode and the second electrode are finely shaved, so that oxidation of the electrodes may proceed rapidly through the damaged parts. In this case, the performance of electrolysis decreases, and as a result, the durability of the electrodes decreases. Therefore, to achieve high electrolysis efficiency without significantly reducing the durability of the electrodes, the length, area, and interval between the first electrode and the second electrode need to be appropriately adjusted.

Generally, various methods for generating microbubbles are known. The electrolysis tube according to the present invention generates microbubbles by underwater discharge using electrode plates. Underwater discharge uses a strong discharge effect to electrolyze water molecules into hydrogen ions and oxygen ions, and performs the electrolysis continuously to be used for skin cleansing. As bubbles generated by underwater discharge are smoothly discharged through the space between the positive electrode and the negative electrode, underwater discharge occurs in the entire area between the two electrodes. Due to underwater electrolysis in which active electrolysis of water molecules can occur, microbubbles are generated.

Electrolysis refers to the process of forcibly separating a substance that does not naturally separate into anions and cations in an aqueous solution state into anions and cations using electrical force. In particular, electrolysis of water can generate Brown's Gas, which is a mixture of oxygen and hydrogen in a 1:2 ratio.

The microbubbles generally have a size of 50 µm or less, thus having very small buoyancy, and rise at a slow speed of about 3 mm/min, staying in water for a long period. Due to this, water in which a large amount of microbubbles are generated has a high dissolved oxygen content. Surface tension acts on the surface of microbubbles generated in water, and due to this, the internal pressure (self-pressurization effect) in the shrunken bubbles increases. This action leads to a longer increase in internal pressure. The gas with increased pressure due to microbubble formation can efficiently enter the water, thereby improving the gas dissolution effect. Therefore, microbubbles have a high gas dissolution effect, electrostatically attract organic substances, and have a sterilizing and cleansing effect through the energy generated when they explode, making them suitable for skin cleansing.

Although the microbubbles stay in water longer than general bubbles, the microbubbles must be fully applied to the skin to effectively affect cleansing. Therefore, the interval between the time when microbubbles are generated from the microbubble generator and the time when they are applied to the skin should be minimized. To increase electrical conductivity during electrolysis, there is a need to add an electrolyte such as NaCl, but in the present invention, pure water is electrolyzed through the electrodes.

FIG. 9 is a photograph showing an actual electrode that can be used in an electrolysis tube according to an embodiment of the present invention, and FIG. 10 is a view showing plating layers of an electrode that can be used in an electrolysis tube according to an embodiment of the present invention. As shown in FIGS. 9 and 10, the first electrode (43) and the second electrode (44) mean a positive electrode and a negative electrode, respectively, and the first electrode (43) and the second electrode have a plate shape extending in one direction, and if necessary, may have a mesh structure in which a plurality of openings are formed in the plate electrode.

The first electrode (43) and the second electrode (44) may be electrode plates in which a platinum-based metal (42b) with high catalytic efficiency for electrolysis is plated on titanium metal (42a). To efficiently generate microbubbles, the electrodes (42) are preferably those in which a platinum-based metal (42b) with high electrolysis catalytic effect is plated on titanium metal (42a) with strong corrosion resistance.

The pair of electrodes (42) used in the electrolysis tube (40) may be electrodes in which a platinum-based metal (42b) is plated on a titanium electrode (42a), considering all of the electrolysis catalytic efficiency, reduction in electrode lifespan due to separation of metal ions during discharge, and possibility of harmful chemical changes during oxidation. Furthermore, the efficiency of the electrodes (42) can be further maximized with a mesh structure in which a plurality of openings are formed in the plate shape.

As shown in FIG. 10, the electrodes (42) may be plated in two or more layers for durability. That is, titanium metal (42a) may be secondarily plated on the electrode in which the platinum-based metal (42b) is plated on the titanium metal (42a). The titanium metal (42a) and the platinum-based metal (42b) may be plated to the same thickness. The thickness (C) of the plating of the titanium metal (42a) and the platinum-based metal (42b) is preferably between 0.1 µm and 10 µm. If the plating thickness is outside this range, there is a problem that the total surface area decreases in a mesh structure electrode of the same area, and the economic feasibility of the product also decreases.

The electrolysis tube (40) includes: the moving tube (41), which is the moving path of the water supplied to the inlet (46); and the pair of electrodes (42) that apply voltage to the moving water to progressively electrolyze the water moving through the moving tube (41) to generate microbubbles. Therefore, the water supplied from the water tank is progressively electrolyzed until the end when it is discharged through the outlet (48), continuously generating microbubbles.

The electrolysis tube (40) according to the present invention allows the water withdrawn from the water tank (20) by the pump (30) to be immediately (instantly) electrolyzed inside the moving tube (flow path tube) (41) , in which the water is moving to the practitioner's skin, continuously and abundantly generating microbubbles. Therefore, the electrolysis tube (40) according to the present invention does not require a separate bubble generation chamber.

In addition, electrodes are installed facing each other at close intervals in the narrow moving tube (41) (flow path tube) of the electrolysis tube (40). Therefore, as microbubbles are generated, water is vaporized by local discharge heat, and the bubbles formed at this time cause discharge even by a weak electric field, further inducing the generation of microbubbles.

The microbubble cleanser having an electrolysis tube according to the present invention can detect the amount of liquid passing through the electrolysis tube (40) in various ways. Through this, overcurrent or overheating can be prevented in advance. For example, using a capacitance sensor or an I-V Converter, the amount of water or the presence or absence of water can be measured.

The vibration driving unit (50) can vibrate the vibration cleansing unit (70) by conventional methods such as motor driving or ultrasonic driving. The vibration driving unit (50) includes a built-in battery (52) located inside the housing (10) and a vibration motor (54) connected to the upper part of the built-in battery (52). The vibration driving unit (50) may be coupled inside the housing (10) by a predetermined bracket.

As the vibration motor (54) is rotated by the battery (52), vibration is generated. The vibration motor (54) is preferably rotated by 180°. The intensity of vibration can be adjusted according to the rotation speed of the vibration motor (54). The vibration cleanser according to the present invention operates by the built-in battery (52) and can obtain cleansing effects and vibration effects.

The control unit (60) is electrically connected to the battery (52) and controls components (for example, pump (30), electrolysis tube (40), vibration driving unit (50), vibration cleansing unit (70), etc.) according to a set program. The control unit (60) can prevent overcurrent or overheating by setting voltage, current, operation time, etc., and can control (adjust) the generation of microbubbles.

The vibration cleansing unit (70) is located at one end of the housing (10), for example, may have a cross-sectional shape substantially similar to the housing (10) to cover the open one surface of the housing (10). The vibration cleansing unit (70) is a part that cleanses the skin by friction in contact with the user's skin, and may have a substantially flat surface shape to contact the user's skin over a wide area.

The vibration cleansing unit (70) further includes a vibration plate (not shown) that can vibrate in the vertical direction and a cleansing pad (72) formed on the upper part of the vibration plate.

The vibration plate is not particularly limited in its shape or material, but may be made of a flexible material such as rubber or silicone.

The cleansing pad (72) vibrates in the vertical direction while contacting the skin, and cleanses the skin by friction with the skin. The cleansing pad (54) may be a brush, protrusion, sponge, cleansing towel, cleansing pad, etc., for cleansing the skin by contact and friction with the skin.

The cleansing pad (72) may be integrally formed with the vibration plate, adhered by an adhesive, or coupled by a conventional coupling method such as physical fitting. A through-hole through which the outlet (44) of the electrolysis tube (40) is exposed to the outside is formed in the cleansing pad (72) and the vibration plate of the vibration cleansing unit (70).

The electrolysis tube (40) according to the present invention has a pair of electrodes (42) positioned close to each other oppositely, so miniaturization is easy, and it can be suitably used in small-sized skin beauty devices. In addition, the microbubble cleanser according to the present invention can generate abundant microbubbles by electrolyzing the water applied to the user's skin using an ultra-small electrolysis tube (40) and a metering pump (30). Water rich in microbubbles is supplied to the user's skin and used for skin cleansing.

Although the present invention has been described with reference to the accompanying drawings and exemplary embodiments, the present invention is not limited to the contents shown in the drawings and the above-described embodiments. In the following claims, reference numerals are indicated for ease of understanding, but the scope of the following claims is not limited to the reference numerals and the contents shown in the drawings, and should be interpreted to encompass all modifications, equivalent configurations, and functions of the exemplary embodiments.

### [Industrial applicability]

The present invention provides a microbubble cleanser having an electrolysis tube.

## Claims

1. A microbubble cleanser comprising:
a water tank (20) coupled to one end of a housing (10) and containing pure water, which is a medium in which microbubbles are generated and supplied;
a pump (30) located at the upper part of the water tank (20), withdrawing the water contained in the water tank (20), and transferring the withdrawn water at a predetermined pressure so that the withdrawn water is sprayed onto the user's skin through an electrolysis tube (40); and
an electrolysis tube (40) located at one end of the pump (30), electrolyzing the water supplied from the water tank (20) by the pump (30) to generate microbubbles, and supplying the water containing the generated microbubbles to the user's skin,
wherein the electrolysis tube (40) includes an inlet (46) through which water is supplied, a moving tube (41) through which the water supplied through the inlet (46) moves a predetermined distance, an outlet (48) through which the water moved through the moving tube (41) is discharged, and a pair of electrodes (42) consisting of a first electrode (43) and a second electrode (44) positioned opposite each other on both sides of the moving path of the water moving through the moving tube (41), and applying a voltage to the moving water to progressively electrolyze the water moving through the moving tube (41) to generate microbubbles.

2. The microbubble cleanser according to claim 1, wherein the water supplied to the inlet (46) is introduced into one end of the electrodes (42), progressively electrolyzed while passing between the pair of electrodes (42), and flows out to the other end of the electrodes (42).

3. The microbubble cleanser according to claim 1, further comprising a vibration cleansing unit (70) that cleanses the skin by friction in contact with the user's skin and a vibration driving unit (50) that vibrates the vibration cleansing unit (70).

4. The microbubble cleanser according to claim 3, wherein the vibration driving unit (50) includes a vibration motor (54) that rotates by 180°.

5. The microbubble cleanser according to claim 1, wherein the housing (10) further includes a through-hole (14) through which the outlet (48) of the electrolysis tube (40) is exposed to the outside so that water containing microbubbles generated in the electrolysis tube (40) can be discharged to the user's skin.
